(19) Europäisches Patentamt / European Patent Office / Office européen des brevets

(11) **EP 2 686 686 B1**

(12) **FASCICULE DE BREVET EUROPEEN**

(45) Date de publication et mention de la délivrance du brevet:
**14.10.2015 Bulletin 2015/42**

(21) Numéro de dépôt: **12712744.7**

(22) Date de dépôt: **12.03.2012**

(51) Int Cl.:
**G01N 33/68** *(2006.01)*

(86) Numéro de dépôt international:
**PCT/IB2012/051156**

(87) Numéro de publication internationale:
**WO 2012/123886 (20.09.2012 Gazette 2012/38)**

(54) **MÉTHODE POUR CARACTÉRISER L'ORIGINE ET/OU L'ÉTAT DE CELLULES PATHOLOGIQUES OU SAINES ET SES APPLICATIONS EN BIOLOGIE**

VERFAHREN ZUR CHARAKTERISIERUNG DES URSPRUNGS UND/ODER DES ZUSTANDES VON ERKRANKTEN ODER GESUNDEN ZELLEN UND SEINE VERWENDUNG IN DER BIOLOGIE

METHOD FOR CHARACTERISING THE ORIGIN AND/OR CONDITION OF DISEASED OR HEALTHY CELLS, AND USES THEREOF IN BIOLOGY

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **14.03.2011 FR 1152047**

(43) Date de publication de la demande:
**22.01.2014 Bulletin 2014/04**

(73) Titulaire: **Université de Nantes
44035 Nantes Cedex 1 (FR)**

(72) Inventeurs:
- **TEA, Illa**
  **F-44000 Nantes (FR)**
- **MARTINEAU, Estelle**
  **F-44640 Rouans (FR)**
- **GIRAUDEAU, Patrick**
  **F-44300 Nantes (FR)**
- **AKOKA, Serge**
  **F-44700 Orvault (FR)**
- **NION, Sophie**
  **F-44300 Nantes (FR)**

(74) Mandataire: **Cabinet Armengaud Aîné
3, avenue Bugeaud
75116 Paris (FR)**

(56) Documents cités:
- **WILKINSON MICHAEL J ET AL: "Age-related variation in red blood cell stable isotope ratios (delta13C and delta15N) from two Yupik villages in southwest Alaska: a pilot study.", INTERNATIONAL JOURNAL OF CIRCUMPOLAR HEALTH FEB 2007 LNKD- PUBMED:17451132, vol. 66, no. 1, février 2007 (2007-02), pages 31-41, XP002652768, ISSN: 1239-9736**
- **MORRISON DOUGLAS J ET AL: "Authenticating production origin of gilthead sea bream (Sparus aurata) by chemical and isotopic fingerprinting", LIPIDS, SPRINGER, US, vol. 42, no. 6, 1 janvier 2007 (2007-01-01), pages 537-545, XP009110958, ISSN: 0024-4201, DOI: DOI: 10.1007/S11745-007-3055-3**
- **SHCHEPINOV MIKHAIL S: "Do "heavy" eaters live longer?", BIOESSAYS : NEWS AND REVIEWS IN MOLECULAR, CELLULAR AND DEVELOPMENTAL BIOLOGY DEC 2007 LNKD-PUBMED:18027392, vol. 29, no. 12, décembre 2007 (2007-12), pages 1247-1256, XP002652769, ISSN: 0265-9247**
- **HARRISON S M ET AL: "Tissue turnover in ovine muscles and lipids as recorded by multiple (H, C, O, S) stable isotope ratios", FOOD CHEMISTRY, ELSEVIER LTD, NL, vol. 124, no. 1, 1 janvier 2011 (2011-01-01), pages 291-297, XP027183497, ISSN: 0308-8146 [extrait le 2010-06-15]**

**Description**

L'invention a pour objet une méthode pour caractériser l'origine et/ou l'état de cellules pathologiques, notamment cancéreuses. Elle a également pour objet les applications de cette méthode en biologie, comprenant le domaine de la santé du diagnostic et de la recherche.

La caractérisation des cellules, notamment de tumeurs cancéreuses, repose principalement sur la diversité morphologique des cellules.

D'autres techniques, basées sur le phénotype moléculaire (ADN, ARN, et protéines), telles que les puces à ADN et à protéines, se sont développées.

Ces techniques peuvent aider à comprendre les mécanismes complexes de la pathologie puisqu'elles permettent d'identifier des milliers de gènes et de protéines impliqués au sein d'une cellule.

Cependant, leur mise en oeuvre nécessite des préparations d'échantillons et une analyse des résultats très longue, pas toujours discriminante pour différencier des cellules.

Il est de plus difficile de les utiliser en routine car souvent les résultats ont besoin d'être confirmés gène par gène ou protéine par protéine par d'autres techniques de validation des phénotypes moléculaires les plus courants, comme la Réaction en Chaîne par Polymérase quantitative (qPCR) après transcription inverse pour les profils d'expression génique et immunoblot ou immunocyto/histochimie pour les protéines.

De telles méthodes sont souvent aléatoires, non précises, longues à mettre en oeuvre, fastidieuses et coûteuses.

Wilkinson et al. (International Journal of Circumpolar Health, Feb 2007, vol. 66, no.1) décrivent l'utilisation des rapports isotopiques $^{13}$C et $^{15}$N comme biomarqueurs de régimes alimentaires selon l'âge, pour caractériser l'état de cellules saines.

Morrison et al. (Lipids, vol. 42, no.6, 1 janvier 2007) décrivent l'authentification de l'origine des poissons (page 538, colonne 2, « Samples ») par analyse isotopique d'une fraction lipidique, telle que l'huile de poisson (abstract).

Shchepinov (Bioessays. 2007 Dec;29(12):1247-56) décrit que les plantes d'origine marine ont une quantité de $^{13}$C supérieure à celles de plantes terrestre.

Les travaux des inventeurs ont alors porté sur la recherche d'une méthode analytique simple permettant notamment de différencier avec précision des cellules, en particulier les tumeurs cancéreuses, de différencier également les effets d'une substance sur les cellules pathogènes, dans le cadre d'une évaluation pré-clinique, d'obtenir une analyse rapide, peu coûteuse et facile à mettre en oeuvre afin de permettre son usage dans un laboratoire de routine.

Les résultats obtenus ont montré que les mesures des variations de la composition isotopique de certains éléments des cellules dépendent bien des effets isotopiques associés aux processus biochimiques et constituent une signature isotopique permettant de caractériser une cellule pathologique, spécialement une cellule cancéreuse, ou à titre comparatif une cellule saine.

L'invention a donc pour but de fournir une nouvelle méthode pour caractériser l'origine et/ou l'état de cellules pathologiques.

Elle vise en particulier l'identification de cellules pathologiques, en particulier cancéreuses, basée sur la mesure de variations de la composition isotopique de certains éléments présents dans les cellules.

L'invention vise également à fournir des moyens pour l'étude des perturbations métaboliques de ces cellules.

Elle porte en outre sur les applications de cette méthode pour l'évaluation de l'effet d'un traitement thérapeutique et pour l'élaboration de bases de données.

La méthode selon l'invention, pour caractériser l'origine et/ou l'état de cellules pathologiques, est caractérisée en ce qu'elle comprend la mesure des variations isotopiques en abondance naturelle d'éléments des cellules, ou d'extraits de cellules, dont les teneurs sont modifiées en situation pathologique la mesure des variations isotopiques de $^{15}$N et $^{13}$C avec un Spectromètre de Masse de Rapports Isotopiques (SMRI en abrégé), avantageusement avec un Analyseur Elémentaire couplé à un Spectromètre de Masse de Rapports Isotopiques (AE-SMRI en abrégé)

Par extrait de cellules, on entend des cellules lyophilisées et/ou des fractions de ces cellules.

Dans un mode préféré de réalisation de l'invention, les variations isotopiques mesurées sont celles de $^{15}$N et $^{13}$C.

Les teneurs isotopiques de ces éléments sont spécialement exprimées par le rapport isotopique R des courants ioniques de la fraction de l'isotope le plus lourd sur l'isotope le plus léger. Il s'agit tout particulièrement des rapports isotopiques $^{15}$N/$^{14}$N et $^{13}$C/$^{12}$C.

Selon une disposition supplémentaire, les variations isotopiques en abondance naturelle de $^{2}$H et/ou de $^{18}$O et/ou de $^{34}$S sont également mesurées.

Ces différentes mesures permettent d'obtenir rapidement, le plus généralement en une dizaine de minutes seulement, une empreinte ou signature isotopique pour chaque type cellulaire étudié.

Dans un mode préféré de réalisation de l'invention, la méthode ci-dessus est caractérisée en ce qu'elle comprend au moins l'une des étapes suivantes :

- l'introduction des extraits de cellules avec un flux contenant de l'oxygène dans le four à combustion d'un AE-SMRI aux fins d'oxydation et/ou de réduction pour la transformation quantitative des cellules en gaz,
- l'élimination de l'eau formée et la séparation des gaz
- l'introduction des gaz dans le SMRI pour introduire des ions moléculaires recueillis dans des collecteurs
- l'établissement des spectres des courants ioniques des isotopomères des éléments isotopiques à mesurer,
- la teneur isotopique des cellules étudiées étant exprimée par le rapport isotopique R des courants ioniques de la fraction de l'isotope le plus lourd sur l'isotope le plus léger et, si souhaité,
- la comparaison des valeurs obtenues avec celle d'un gaz de référence étant exprimée en delta pour mille.

Avantageusement, la méthode de l'invention comprend en outre l'introduction des extraits des cellules dans un chromatographe en phase gazeuse pour séparer les molécules, puis dans un four de combustion et réduction pour la transformation quantitative des cellules en gaz, puis après l'élimination de l'eau, l'introduction des gaz dans le SMRI comme indiqué ci-dessus.

Les dispositions qui précèdent, en partie ou dans leur totalité, sont appliquées à une fraction cellulaire, en particulier une fraction protéique, ou aux acides aminés et/ou aux métabolites polaires intracellulaires ou non ou, selon une autre variante, aux lipides extraits des cellules.

Le matériau étudié est constitué par des cellules provenant d'un tissu ou d'un milieu biologique, tel que le sang ou l'urine, ou par des cellules d'une lignée cellulaire.

Lesdites cellules sont des cellules pathologiques, c.-à-d. des cellules provenant de tout type de pathologie, notamment des cellules cancéreuses. Des cellules saines peuvent être utilisées pour faire des comparaisons.

Il est ainsi possible de disposer d'une empreinte isotopique propre à chaque tumeur, pouvant servir de bio-marqueur, et permettant de différencier avec précision des cellules tumorales, des cellules pathologiques et d'évaluer les effets d'une action thérapeutique sur ces cellules, dans le cadre d'une évaluation pré-clinique.

La facilité de mise en ouvre de la méthode de l'invention, la rapidité de son exécution et son faible coût constituent des avantages pour une utilisation en routine.

Les applications de cette méthode sont nombreuses. Parmi les secteurs concernés, on citera :

- les secteurs de la recherche en biologie, biochimie au niveau cellulaire ou tissulaire (caractérisation des cellules et tissus, étude des voies métaboliques, connexions entre l'empreinte isotopique et le processus de la cellule cancéreuse)
- les secteurs industriels de la pharmacie (évaluation de l'effet thérapeutique d'une substance sur un patient)
- les secteurs de la santé (diagnostic médical adapté, suivi du patient).

La méthode de l'invention possède de nombreux avantages en termes de performances puisque la mesure est reproductible et insensible à la variation biologique (mesures identiques pour des cellules qui ont été divisées plusieurs fois: jusqu'à 48 divisions cellulaires). La mesure peut être facilement comparée avec d'autres laboratoires, étant donné que la déviation isotopique est une mesure relative qui est toujours comparée à une référence internationale. Ainsi les laboratoires possédant un AE-SMRI sont susceptibles d'obtenir une signature unique.

Le coût de l'analyse est dérisoire contrairement aux bio-puces, puisque la préparation d'échantillon et l'analyse sont très simples. Il suffit de récupérer un extrait sec de cellule, de tissu, de sang, d'urine ou autres liquides biologiques, puis de l'introduire dans l'AE-SMRI.

En revanche dans le cas des bio-puces, des étapes d'extraction, de purification de l'ARNm et des protéines de l'échantillon sont nécessaires. L'ARNm est ensuite converti en ADNc par transcription inverse. Les extraits d'ADNc ou de protéines sont ensuite marqués par un fluorochrome de couleurs différentes. Toutes ces étapes complexes de préparations d'échantillons sont des sources d'erreur pour l'analyse. La préparation des bio-puces est aussi longue et fastidieuse, puisqu'elle consiste à immobiliser sur un support solide des sondes (ADN ou anticorps dans le cas des protéines) dont le rôle est de détecter les cibles complémentaires présentes dans le mélange à analyser (ADNc ou protéines). Les résultats sont obtenus après l'analyse d'images à haute résolution qui permet de déceler les gènes ou les protéines dont le taux est modifié au cours de la transformation cancéreuse.

La simplicité de la méthode de l'invention, qui demande très peu de préparation d'échantillons et une analyse par AE-SMRI très fiable constituent des atouts, pour la reproductibilité des mesures, ainsi que pour l'utilisation adaptée de cette méthode dans le domaine de la recherche biologique et clinique.

Le fonctionnement de la méthode de l'invention est en outre plus écologique que celui des bio-puces, puisqu'il ne nécessite pas l'utilisation de produits chimiques. Dans le cas des bio-puces, l'utilisation des tampons d'extraction (sodium dodécyl sulfate, mercaptoéthanol et autres détergents...) et du marquage parfois radioactif ou fluorescent sont souvent nocifs pour le manipulateur. Les bio-puces sont jetables, ce qui produit des déchets.

L'invention vise également l'application de la méthode définie ci-dessus, pour discriminer un type cellulaire et/ou des sous-types, identifier l'état pathologique ou sain des cellules étudiées, déterminer les perturbations biologiques liées aux mesures effectuées, et/ou évaluer l'efficacité d'une action thérapeutique et/ou pour constituer une base de données servant de référentiel pour identifier le type et le stade de la pathologie et permettre ainsi le traitement adapté à celle-ci.

Ces bases de données entrent également dans le champ de l'invention. Elles sont caractérisées en ce qu'elles renferment les données relatives aux empreintes isotopiques telles qu'établies selon la méthode définie ci-dessus pour différents types cellulaires et tissus, en particulier cancéreux.

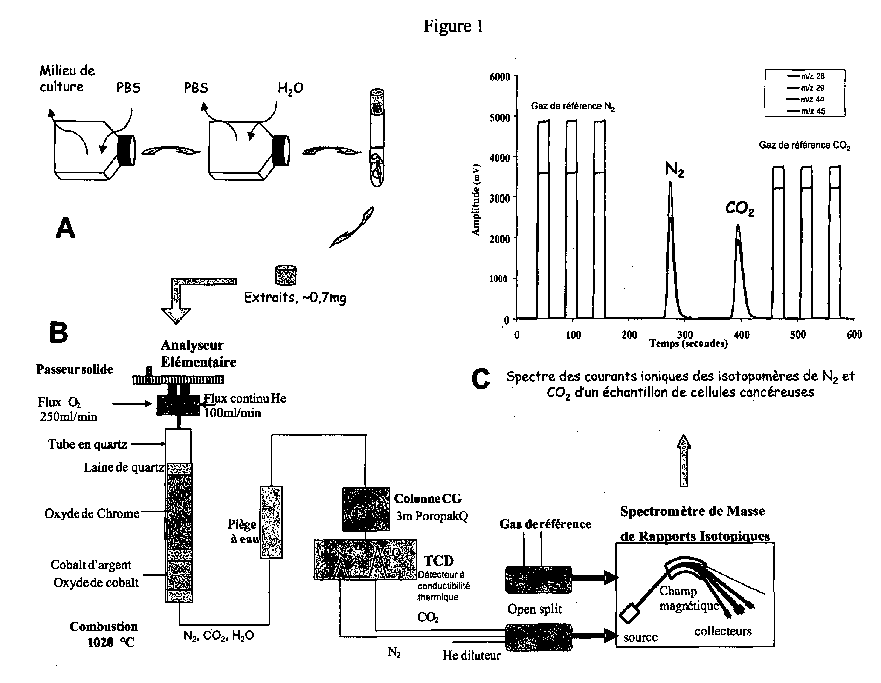

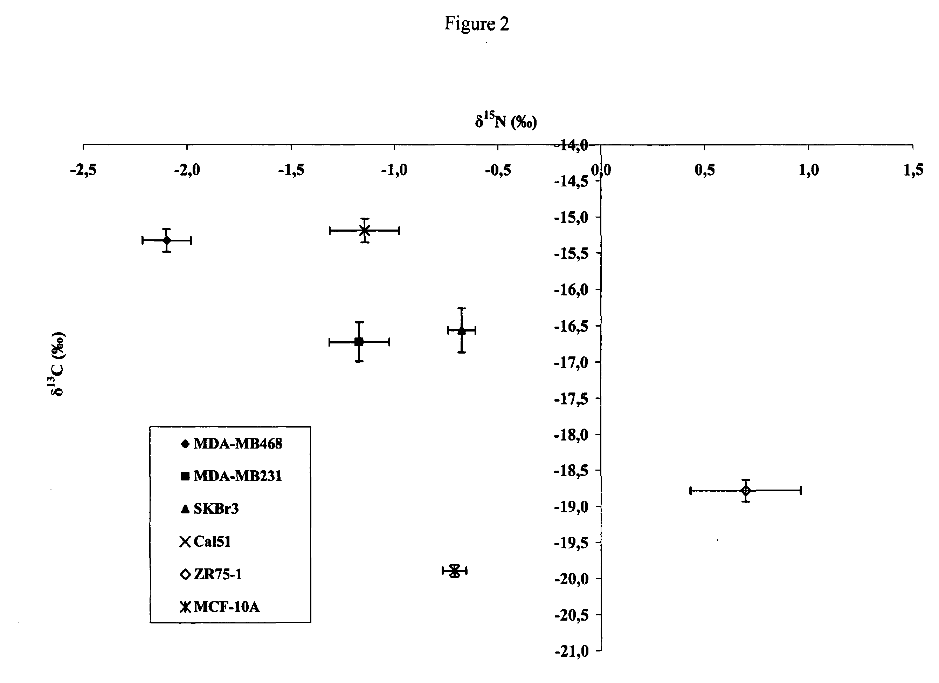

D'autres caractéristiques et avantages de l'invention sont donnés dans les exemples qui suivent et sont illustrés par les résultats donnés dans les Figures 1 et 2, qui représentent respectivement la méthode de l'invention

- la Figure 1, la description schématique des étapes de la méthode de l'invention, permettant d'obtenir l'abondance naturelle des isotopes de l'azote 15 et du carbone 13 par exemple dans des cellules cancéreuses et
- la Figure 2, les valeurs des déviations isotopiques $\delta^{15}N$ et $\delta^{13}C$ des cellules de différentes lignées par AE-SMRI.

Exemple 1 : étude de lignées cellulaires humaines du cancer du sein.

Les lignées cellulaires étudiées sont les lignées MDA MB-468, MDA MB-231, SkBr3, Cal 51, ZR 75-1 et MCF-10A et sont décrites dans le tableau I.

**Tableau I :** Caractéristiques des lignées du cancer du sein étudiées. Le nom et la numérotation utilisée par l'ATCC (American Type Culture Collection) sont donnés pour chaque lignée. Les signes + et - traduisent la présence et l'absence des récepteurs correspondants aux récepteurs des oestrogènes (ER), des progestérones (PR) et à un des membres des récepteurs à tyrosines kinases (ErbB), nommé HER2.

| Lignées cellulaires | N°ATCC | ER | PR | HER2 | Pathologie |
|---|---|---|---|---|---|
| MDA-MB468 | HTB-132 | - | - | - | métastase d'adénocarcinome |
| MDA-MB231 | HTB-26 | - | - | + | métastase d'adénocarcinome |
| SKBr3 | HTB-30 | - | - | ++ | adénocarcinome |
| Cal51 | DSZM-ACC302 | - | - | - | adénocarcinome |
| ZR75-1 | CRL-1500 | + | - | - | carcinome canalaire invasif |
| MCF-10A | CRL-10317 | - | - | - | maladie fibrokystique (non cancéreuse) |

Toutes les lignées cellulaires sont cultivées dans 15 ml de milieu de culture nutritif dans une flasque d'une surface de 75 cm$^2$.

Pour toutes les lignées cellulaires, excepté pour la lignée MCF-10A, le milieu contient du DMEM (Dulbecco's Modified Eagle Medium) supplémenté avec 10% de sérum foetal bovin (FBS), 1% pénicilline-streptomycine (Invitrogen).

Pour les cellules de la lignée MCF-10A, le milieu utilisé est le DMEM F12 (Invitrogen) contenant 5% de Sérum de cheval, 20 ng/ml d'EGF (Epidermal Growth Factor), 2mg/ml de toxine cholérique, 0,01 mg/ml d'insuline, 0,5 $\mu$g/ml d'hydrocortisone, 1% de L-Glutamine Glutamax, 1 % de pénicilline-streptomycine, 1% d'Hépès (produits Sigma).

Les flasques contenant les cellules sont placées dans un incubateur à humidité et atmosphère contrôlées (5% $CO_2$), à une température de 37°C. Lorsque les cellules sont confluentes (à peu près 2 000 000 de cellules), le milieu de culture est éliminé. Les cellules sont rincées 2 fois avec 5 ml de tampon phosphate salin (PBS) de pH=7,4 afin d'éliminer entièrement le milieu nutritif. Puis les cellules sont récupérées et détachées à l'aide d'un grattoir dans 3 ml d'eau distillée. Elles sont ensuite stockées à -20°C puis lyophilisées (Fig. 1A).

Environ 0,7 mg d'extrait sec de cellules est ensuite pesé avec une balance de précision 10$^{-5}$g (Ohaus Discovery DV215CD, Pine Brook, New Jersey, USA) dans des capsules en étain (tin capsules for solids « light » 5 x 9mm, Thermo Fisher Scientific, Brême, Allemagne), pour permettre l'analyse dans un Analyseur Elémentaire (AE ; Flash EA 1112HT, Thermo Fisher Scientific, Brême, Allemagne) couplé via une interface conflo (Finnigan Conflo III, Thermo Fisher Scientific, Brême, Allemagne) à un Spectromètre de Masse de Rapports Isotopiques (SMRI ; IRMS Delta V Advantage, Thermo Fisher Scientific, Brême, Allemagne).

La capsule en étain contenant les cellules est introduite grâce à un flux d'hélium et d'oxygène dans le four de combustion de l'AE à 1020°C. A cette température, l'étain permet par sa sublimation, un transfert d'énergie à l'échantillon de cellules qui est oxydé très rapidement. La composition du four de combustion (oxyde de chrome, cuivre réduit, oxyde de cobalt et cobalt d'argent) et la quantité d'oxygène introduite permettent de transformer quantitativement l'échantillon de cellules en gaz $N_2$, $CO_2$ et $H_2O$.

Les gaz passent au travers d'un piège à anhydrone (perchlorate de magnésium) retenant l'eau.

Les gaz $N_2$ et $CO_2$ sont ensuite séparés par une colonne de chromatographie, puis introduits successivement dans le SMRI (Fig. 1 B).

Le $N_2$ et $CO_2$ produits au cours de la combustion de l'échantillon arrivent dans la source du SMRI où ils sont ionisés par impact électronique à 120 eV(électron Volt), sous une pression de $10^{-6}$ mbar.

Les ions moléculaires produits ($N_2^{+\cdot}$ et $CO_2^{+\cdot}$ sont ensuite accélérés par un potentiel de 3 kV et sont projetés dans un champ magnétique uniforme. Ces ions sont ensuite déviés par ce champ magnétique de 0.75 Tesla et sont recueillis simultanément dans des collecteurs (cages de Faraday). Les cages de Faraday sont reliées à des amplificateurs et les courants produits sont proportionnels à la quantité respective de chaque espèce d'ions collectés.

Un spectre des courants ioniques des isotopomères de $N_2$ et $CO_2$ est obtenu en 10 minutes (Fig. 1 C).

Pour l'azote, il s'agit des isotopomères de masses atomiques 28, 29, 30 correspondant respectivement aux isotopes du diazote $^{14}N^{14}N$, $^{14}N^{15}N$, $^{15}N^{15}N$.

De même pour le carbone, les isotopomères de masses atomiques 44, 45 et 46 sont détectés et correspondent respectivement aux isotopes du dioxyde de carbone $^{12}C^{16}O_2$, $^{13}C\ ^{16}O_2$ ou $^{12}C^{17}O^{16}O$, $^{12}C^{17}O_2$.

La teneur isotopique de l'échantillon est exprimée par le rapport isotopique R des courants ioniques qui est la fraction de l'isotope le plus lourd sur l'isotope le plus léger soit $^{13}C/^{12}C$ et $^{15}N/^{14}N$.

L'utilisation d'une référence permet de comparer le rapport isotopique de l'échantillon à celui de la référence et d'obtenir ainsi une grande précision des résultats qui sont exprimés dans l'échelle relative delta $\delta$(‰) :

$$\delta\ (‰) = [(R_{\text{échantillon}} - R_{\text{référence}})/ R_{\text{référence}}] \times 1000$$

Les références internationales repérées dans la formule par $R_{\text{référence}}$ sont le carbonate Vienna Pee Dee Belemnite (VPDB) pour le $\delta^{13}C$ ($R_{\text{référence}}$ = 0,0112372) et l'azote atmosphérique pour le $\delta^{15}N$ ($R_{\text{référence}}$ = 0,0036765).

Une valeur positive signifie une teneur plus élevée en isotope lourd que la référence, c'est-à-dire un enrichissement ; alors qu'une valeur négative exprime une teneur moins élevée en isotope lourd que la référence c'est-à-dire un appauvrissement.

Comme standard de travail, l'acide glutamique a été utilisé ; ses $\delta^{13}C$ et $\delta^{15}N$ sont connus et stables, respectivement -27,48‰ $\pm$ 0,05 et -4,80‰ $\pm$ 0,08.

Deux capsules d'acide glutamique ont été placées tous les 5 échantillons afin de contrôler une éventuelle dérive de l'appareil au cours de la série de mesure.

La linéarité de l'appareil est de $\pm$0,06‰ ; la précision est de $\pm$0,02‰ pour $\delta^{13}C$ et pour $\delta^{15}N$ (données de Thermo Fisher Scientific).

Le processus de recueil de données a été effectué à l'aide du logiciel d'acquisition Isodat NT 2.5 (Thermo Fisher Scientific, Brême, Allemagne) qui inclut la correction automatique pour la fraction $^{17}O$ de la masse 45 par la correction de Craig. Les valeurs de pourcentages de carbone (%C) et d'azote (%N) sont calculées à partir du rapport d'aire sous la courbe du pic de l'échantillon sur le pic du standard de travail, l'acide glutamique.

Chaque extrait cellulaire a fait l'objet de 2 analyses. Le résultat pris en compte est la moyenne des deux résultats de mesure. Pour chaque lignée, l'analyse a été effectuée sur 3 à 6 échantillons d'une division cellulaire, afin d'avoir une valeur isotopique représentative de la lignée.

Statistiques

Les données ont été exportées du logiciel d'acquisition vers un tableur Microsoft Excel 2003 où ont été effectuées les moyennes et écarts-types des $\delta^{13}C$ et $\delta^{15}N$ de chaque lignée cellulaire, ayant subi plusieurs divisions cellulaires.

Résultats

Les résultats obtenus (valeurs des déviations isotopiques $\delta^{15}N$ et $\delta^{13}C$ et des pourcentages d'azote %N et de carbone %C) pour les lignées étudiées sont rassemblés dans le tableau II et représentés dans la figure 2.

**Tableau II :** Valeurs des déviations isotopiques ($\delta^{15}$N et $\delta^{13}$C) et des pourcentages d'azote et de carbone (%N et %C), mesurées par AE-SMRI, dans les échantillons de cellules de différentes lignées étudiées en fonction du nombre de divisions cellulaires (NDC) et du nombre de jours de croissance (NJC).

| Lignées cellulaires | NDC | NJC | $\delta^{15}$N (‰) | $\delta^{13}$C (‰) | %N | %C |
|---|---|---|---|---|---|---|
| MDA-MB468 | 26 | 2 | -2.06 | -15.56 | 7.36 | 29.45 |
| | 31 | 4 | -2.28 | -15.28 | 8.67 | 33.53 |
| | 36 | 3 | -2.03 | -15.1 | 8.02 | 32.91 |
| | 39 | - | -2.14 | -15.33 | 8.24 | 33.35 |
| | 42 | 4 | -2.15 | -15.26 | 7.77 | 31.7 |
| | 48 | 2 | -1.94 | -15.44 | 7.79 | 31.5 |
| MDA-MB231 | 2 | 2 | -1.16 | -16.59 | 5.21 | 21.69 |
| | 3 | 4 | -0.97 | -17.12 | 5.33 | 24.84 |
| | 5 | 3 | -1.3 | -16.53 | 5.31 | 22.53 |
| | 6 | 4 | -1.25 | -16.65 | 5.67 | 22.72 |
| SKBr3 | 19 | 4 | -0.69 | -16.92 | 5.13 | 22.41 |
| | 20 | 3 | -0.64 | -16.6 | 4.05 | 16.12 |
| | 21 | 3 | -0.62 | -16.02 | 5.06 | 19.94 |
| | 22 | 3 | -0.6 | -16.51 | 4.86 | 19.64 |
| | 27 | 3 | -0.74 | -16.58 | 6.26 | 24.41 |
| | 31 | 3 | -0.76 | -16.74 | 4.01 | 18.76 |
| Cal51 | 3 | 3 | -0.95 | -15.24 | 6.59 | 28.29 |
| | 4 | 2 | -1.14 | -15.42 | 6.25 | 26.79 |
| | 7 | 2 | -1.28 | -15.2 | 7.38 | 29.88 |
| | 6 | 3 | -1.01 | -14.96 | 7.52 | 30.95 |
| | 6 | 3 | -1.34 | -15.15 | 7.29 | 29.16 |
| ZR75-1 | 4 | 3 | 0.99 | -18.96 | 2.36 | 12.92 |
| | 5 | 3 | 0.47 | -18.68 | 1.97 | 11.37 |
| | 6 | 4 | 0.63 | -18.73 | 1.76 | 12.38 |
| MCF-10A | 5 | | -0.75 | -19.83 | 3.5 | 20.16 |
| | 5 | | -0.67 | -19.95 | 3.65 | 20.97 |

Les valeurs de $\delta^{15}$N et $\delta^{13}$C ont été mesurées pour chaque lignée à divers nombres de divisions cellulaires et de jours de croissance. Les écarts-types obtenus pour chaque lignée cellulaire en fonction du nombre de divisions cellulaires et du nombre de jours de croissance étaient inférieurs à 0,3‰ pour les $\delta^{15}$N et $\delta^{13}$C (Fig. 2). Ces résultats montrent l'absence de fractionnement isotopique (tableau II) induit lors de la croissance cellulaire et du nombre de divisions cellulaires, condition *sine qua non* pour s'assurer que la discrimination isotopique provient uniquement de la cellule elle-même. De la même façon les teneurs isotopiques des milieux nutritifs utilisés pour la croissance de chaque lignée ont été mesurées régulièrement. Elles étaient identiques pour les milieux nutritifs des lignées tumorales ($\delta^{15}$N = 3,98 ± 0,18‰ et $\delta^{13}$C = -12,91 ± 0,13‰). Concernant le milieu utilisé pour la croissance des MCF-10A qui est une lignée non tumorale nécessitant un milieu particulier, la teneur isotopique était aussi constante avec un $\delta^{15}$N = 2,39‰ ± 0,10 et un $\delta^{13}$C = -19,39‰ ± 0,01). Les résultats montrent l'absence de fractionnement isotopique induit par le milieu de culture. Dans ces conditions de culture, les résultats sont très satisfaisants au regard des valeurs des $\delta^{15}$N et $\delta^{13}$C obtenues sur les cellules des différentes lignées, respectivement comprises entre -2,28 et +0,99 ‰ et entre -19,95 et -15,1 ‰ (Fig. 2).

Les valeurs de $\delta^{15}$N et $\delta^{13}$C sont représentatives de chaque lignée. En effet, la variation enregistrée sur 6 lignées cellulaires, cultivées et prélevées à des périodes différentes, ne dépasse pas 0,3‰. De même, les écart-types enregistrés pour les milieux nutritifs utilisés pour chaque lignée sont négligeables. Ces résultats montrent que l'analyse

isotopique simultanée du $^{15}$N et du $^{13}$C réalisée sur chaque lignée constitue une signature caractéristique du type de cancer.

L'invention fournit ainsi un procédé simple et peu coûteux pour identifier une cellule, en particulier pour différencier les tumeurs cancéreuses et évaluer un effet thérapeutique sur ces tumeurs.

## Revendications

1. Méthode pour caractériser l'état pathologique de cellules, **caractérisée en ce qu'**elle comprend la mesure des variations isotopiques en abondance naturelle d'éléments des cellules dont les teneurs sont modifiées en situation pathologique avec un Spectromètre de Masse de Rapports Isotopiques (SMRI en abrégé), avantageusement avec un Analyseur Elémentaire couplé à un Spectromètre de Masse de Rapports Isotopiques (AE-SMRI en abrégé)

2. Méthode selon la revendication 1, **caractérisée en ce que** les variations isotopiques mesurées sont celles de $^{15}$N et $^{13}$C.

3. Méthode selon la revendication 2, **caractérisée par** la détermination de rapports isotopiques $^{15}$N/$^{14}$N et $^{13}$C/$^{12}$C.

4. Méthode selon l'une quelconque des revendications 1 à 3, **caractérisée en ce qu'**elle comprend en outre la mesure des variations isotopiques en abondance naturelle de $^{2}$H et/ou de $^{18}$O et/ou de $^{34}$S.

5. Méthode selon l'une quelconque des revendications 1 à 4, **caractérisée en ce qu'**elle comprend au moins l'une des étapes suivantes

   - l'introduction des extraits de cellules avec un flux contenant de l'oxygène dans le four à combustion d'un AE-SMRI aux fins d'oxydation et/ou de réduction pour la transformation quantitative des cellules en gaz,
   - l'élimination de l'eau formée et la séparation des gaz
   - l'introduction des gaz dans le SMRI pour produire des ions moléculaires recueillis dans des collecteurs
   - l'établissement des spectres des courants ioniques des isotopomères des éléments isotopiques à mesurer,
   - la teneur isotopique des cellules étudiées étant exprimée par le rapport isotopique R des courants ioniques de la fraction de l'isotope le plus lourd sur l'isotope le plus léger et, si souhaité,
   - la comparaison des valeurs obtenues avec celle d'un gaz de référence étant exprimée en delta pour mille.

6. Méthode selon la revendication 5, **caractérisée en ce qu'**elle comprend en outre l'introduction des extraits des cellules dans un chromatographe en phase gazeuse, pour séparer les molécules, puis dans un four de combustion et réduction pour la transformation quantitative des cellules en gaz, puis après l'élimination de l'eau, l'introduction des gaz dans le SMRI.

7. Méthode selon l'une quelconque des revendications 1 à 6, **caractérisée en ce qu'**elle est appliquée à une fraction cellulaire, en particulier une fraction protéique.

8. Méthode selon l'une quelconque des revendications 1 à 6, **caractérisée en ce qu'**elle est appliquée aux acides aminés et/ou aux métabolites polaires intracellulaires ou non ou aux extraits des cellules.

9. Méthode selon l'une quelconque des revendications 1 à 6, **caractérisée en ce qu'**elle est appliquée aux lipides extraits des cellules.

10. Méthode selon l'une quelconque des revendications 1 à 9, **caractérisée en ce qu'**elle est appliquée aux extraits de cellules provenant d'un tissu ou d'un milieu biologique, tel que le sang ou l'urine, ou aux cellules d'une lignée cellulaire ou directement aux tissus, sang, urine.

11. Méthode selon la revendication 10, caractérisée en que lesdites cellules ou tissus sont cancéreux.

12. Méthode selon la revendication 10, caractérisée en que lesdites cellules ou tissus sont pathologiques.

13. Application de la méthode selon l'une quelconque des revendications 1 à 12, pour discriminer un type cellulaire et/ou des sous-types, identifier l'état pathologique des cellules étudiées, et/ou évaluer l'efficacité d'un traitement thérapeutique et/ou pour constituer une base de données servant de référentiel pour identifier le type et le stade de la pathologie et permettre ainsi le traitement adapté à celle-ci.

**Patentansprüche**

1. Verfahren zur Charakterisierung des Zustandes von erkrankten Zellen, das **dadurch gekennzeichnet ist, dass** es die Messung der Variationen der natürlichen Isotopenhäufigkeit von Elementen der Zellen, deren Gehalt im erkrankten Zustand modifiziert ist, mit einem Isotopenverhältnis-Massenspektrometer (abgekürzt IRMS), vorteilhafterweise mit einem Elementaranalysator in Kombination mit einem Isotopenverhältnis-Massenspektrometer (abgekürzt EA-IRMS) misst.

2. Verfahren gemäß Anspruch 1, das **dadurch gekennzeichnet ist, dass** es sich bei den gemessenen Isotopenvariationen um die von $^{15}N$ und $^{13}C$ handelt.

3. Verfahren gemäß Anspruch 2, das durch die Bestimmung der Isotopverhältnisse $^{15}N/^{14}N$ und $^{13}C/^{12}C$ gekennzeichnet ist.

4. Verfahren gemäß einem beliebigen der Ansprüche 1 bis 3, das **dadurch gekennzeichnet ist, dass** es ferner die Messung der Variationen der natürlichen Isotopenhäufigkeit von $^{2}H$ und/oder $^{18}O$ und/oder $^{34}S$ umfasst.

5. Verfahren gemäß einem beliebigen der Ansprüche 1 bis 4, das **dadurch gekennzeichnet ist, dass** es mindestens einen der folgenden Schritte umfasst

   - die Einführung der Zellenextrakte mit einem Sauerstoff enthaltenden Fluss in den Verbrennungsofen eines EA-IRMS zwecks Oxidation und/oder Reduktion zur quantitativen Umwandlung der Zellen in Gas,
   - die Ausscheidung des gebildeten Wassers und die Abscheidung der Gase
   - die Einführung der Gase in das IRMS, um die molekularen Ionen zu produzieren, die in Kollektoren aufgefangen werden
   - die Erstellung der Spektren der Ionenströme der Isotopomere der zu messenden isotopischen Elemente,
   - der Isotopengehalt der untersuchten Zellen, der durch das Isotopenverhältnis R der Ionenströme der schwersten Fraktion des Isotops zum leichtesten Isotop ausgedrückt wird und, falls gewünscht,
   - der Vergleich der erhaltenen Werte mit dem Wert eines Referenzgases, ausgedrückt als Deltawert in Promille.

6. Verfahren gemäß Anspruch 5, das **dadurch gekennzeichnet ist, dass** es ferner die Einführung der Extrakte der Zellen in einen Gaschromatographen umfasst, um die Moleküle zu trennen, dann in einen Verbrennungs- und Reduktionsofen zur quantitativen Umwandlung der Zellen in Gas, dann nach Ausscheidung des Wassers, die Einführung der Gase in den IRMS.

7. Verfahren gemäß einem beliebigen der Ansprüche 1 bis 6, das **dadurch gekennzeichnet ist, dass** es auf eine Zellfraktion, insbesondere auf eine Proteinfraktion angewendet wird.

8. Verfahren gemäß einem beliebigen der Ansprüche 1 bis 6, das **dadurch gekennzeichnet ist, dass** es auf die Aminosäuren und/oder die polaren oder nicht polaren intrazellulären Metaboliten oder auf die Extrakte der Zellen angewendet wird.

9. Verfahren gemäß einem beliebigen der Ansprüche 1 bis 6, das **dadurch gekennzeichnet ist, dass** es auf die aus den Zellen extrahierten Lipide angewendet wird.

10. Verfahren gemäß einem beliebigen der Ansprüche 1 bis 9, das **dadurch gekennzeichnet ist, dass** es auf die Extrakte von Zellen aus einem Gewebe oder einem biologischen Medium wie Blut oder Urin oder auf die Zellen einer Zelllinie oder direkt auf Gewebe, Blut, Urin angewendet wird.

11. Verfahren gemäß Anspruch 10, das **dadurch gekennzeichnet ist, dass** die besagten Zellen oder Gewebe kanzerös sind.

12. Verfahren gemäß Anspruch 10, das **dadurch gekennzeichnet ist, dass** die besagten Zellen oder Gewebe erkrankt sind.

13. Anwendung des Verfahrens gemäß einem beliebigen der Ansprüche 1 bis 12 zur Unterscheidung eines Zelltyps und/oder der Subtypen, zur Ermittlung des erkrankten Zustands der untersuchten Zellen und/oder zur Bewertung der Wirksamkeit einer Therapie und/oder zur Erstellung einer Datenbank, die als Bezugssystem dient, um den Typ und das Stadium der Krankheit zu ermitteln und somit deren geeignete Behandlung zu ermöglichen.

**Claims**

1. A method for characterizing the condition of diseased cells, **characterized in that** it comprises the measurement of natural abundance isotope variations of elements of cells, the contents of which are modified in a situation of disease, using an isotope-ratio mass spectrometer (abbreviated as IRMS), advantageously using an elemental analyzer coupled with an isotope-ratio mass spectrometer (abbreviated as EA-IRMS).

2. The method as claimed in claim 1, **characterized in that** the isotope variations measured are those of $^{15}$N and $^{13}$C.

3. The method as claimed in claim 2, **characterized by** the determination of $^{15}$N/$^{14}$N and $^{13}$C/$^{12}$C isotope ratios.

4. The method as claimed in any one of claims 1 to 3, **characterized in that** it also comprises the measurement of natural abundance isotope variations of $^{2}$H and/or of $^{18}$O and/or of $^{34}$S.

5. The method as claimed in any one of claims 1 to 4, **characterized in that** it comprises at least one of the following steps:

   - introduction of the cell extracts with a stream containing oxygen into the combustion furnace of an EA-IRMS for the purposes of oxidation and/or reduction for the quantitative conversion of the cells into gas,
   - removal of the water formed and separation of the gases,
   - introduction of the gases into the IRMS in order to produce molecular ions collected in collectors,
   - establishment of the spectra of the ion currents of the isotopomers of the isotopic elements to be measured,
   - the isotopic content of the cells studied being expressed by the isotope ratio R of the ion currents of the fraction of the heaviest isotope to the lightest isotope, and, if desired,
   - the comparison of the values obtained with that of a reference gas being expressed in delta per thousand.

6. The method as claimed in claim 5, **characterized in that** it also comprises the introduction of the cell extracts into a gas chromatograph, in order to separate the molecules, and then into a combustion and reduction furnace for the quantitative conversion of the cells into gas, and then, after removal of the water, the introduction of the gases into the IRMS.

7. The method as claimed in any one of claims 1 to 6, **characterized in that** it is applied to a cell fraction, in particular a protein fraction.

8. The method as claimed in any one of claims 1 to 6, **characterized in that** it is applied to amino acids and/or to polar metabolites which may be intracellular or extracellular or to cell extracts.

9. The method as claimed in any one of claims 1 to 6, **characterized in that** it is applied to lipids extracted from cells.

10. The method as claimed in any one of claims 1 to 9, **characterized in that** it is applied to extracts of cells originating from a tissue or from a biological medium, such as blood or urine, or to cells of a cell line or directly to tissues, blood or urine.

11. The method as claimed in claim 10, **characterized in that** said cells or tissues are cancer cells or cancerous tissues.

12. The method as claimed in claim 10, **characterized in that** said cells or tissues are diseased.

13. The use of the method as claimed in any one of claims 1 to 12, for distinguishing a cell type and/or subtypes, identifying the condition of the cells studied, and/or evaluating the effectiveness of a therapeutic treatment and/or for constituting a database which serves as a reference for identifying the type and the stage of the disease and thus allowing suitable treatment thereof.

Figure 1

Figure 2

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **WILKINSON et al.** *International Journal of Circumpolar Health,* Février 2007, vol. 66 (1
- **MORRISON et al.** *Lipids,* 01 Janvier 2007, vol. 42 (6

- **SHCHEPINOV.** *Bioessays,* Décembre 2007, vol. 29 (12), 1247-56